# EUROPEAN PATENT SPECIFICATION

(11) **EP 2 339 964 B1**
(45) Date of publication and mention of the grant of the patent: **11.07.2018**
(21) Application number: 09759787.6
(22) Date of filing: 29.10.2009
(51) Int. Cl.: A61B 5/103

(54) **A PORTABLE SKIN DIAGNOSIS DEVICE**
TRAGBARE HAUTDIAGNOSEVORRICHTUNG
DISPOSITIF DE DIAGNOSTIC CUTANÉ PORTABLE

(30) Priority: 31.10.2008 FR 0857429; 10.11.2008 US 193247 P
(43) Date of publication of application: 06.07.2011
(73) Proprietor: L'Oréal, 75008 Paris (FR)
(72) Inventor: BAZIN, Roland, F-91570 Bievres (FR); GIRON, Franck, F-77164 Ferrieres En Brie (FR); DEBBASCH, Caroline, F-92200 Neuilly Sur Seine (FR); ARKESTEIJN, Walter, NL-5508 EG Veldhoven (NL)
(74) Representative: Nony
(86) International application number: PCT/IB2009/054811
(87) International publication number: WO 2010/049907

(56) References cited:
- EP-A- 1 321 098
- EP-A1- 0 650 694
- WO-A-01/35827
- WO-A1-99/23945
- DE-A1- 10 240 904

## Description

### Technical Field

The present disclosure relates to skin diagnosis tools.

### Background

Pigmentation is a characteristic of the skin that may have implications in various fields of skin physiology, in particular providing protection with regard to effects from solar radiation.

Pigmentation is also a substantial contributor to skin color, and it may be important to measure pigmentation for this reason, e.g. in the context of recommending which foundation is suited to a person's natural color.

Knowledge of phenomena of hyper- or of hypo-pigmentation and of colored marks is also of interest in cosmetics.

In general, each aspect corresponds to a desire for specific, and typically different compositions. Therefore, there is a desire to measure the effect of one or more compositions on a parameter to be improved.

Likewise, each aspect has its own approach and tools for taking measurements. For example, clinical evaluation using color charts as described in publications EP 1 212 961 and EP 1 212 962; appliances for measuring the color or brightness at a point (Mexameter® by Courage and Khazaka, Chromameter® by Minolta); or multispectral methods such as SIAscope by Astron Clinica or Melafind® by EOS. Further, methods with a full face camera such as Visioface by Courage & Khazaka, Visia® by Canfield, Facial Stage® and DM3® by Moritex, Beau Visage by Astron Clinica or Charm View® type microcameras; and imaging methods that enable acquisition of an image in which colorimetric parameters are measured.

Such appliances and methods may enable obtaining parameters that characterize certain colorimetric characteristics that may not be pertinent to one or more aspects described above, and/or that thus require equipment that is costly and/or difficult to transport (e.g., heavy equipment).

Application WO 2008/062967 describes a skin diagnosis device that incorporates a personal digital assistant (PDA) and a measuring module that includes a plurality of light-emitting diodes (LEDs) capable of emitting at different wavelengths. A camera may observe the skin under light from the LEDs. The device may measure pore size, skin pigmentation, and sebum quality optically.

US 2006/0149151 A1 discloses a measuring device for measuring the color of the skin or of the hair.

EP 1 321 098 describes a method for evaluating the typology of the region extending around the human eye, and an apparatus that can perform the evaluation. This includes a camera and at least one lighting device.

WO 01/35827 describes a system for collecting, storing and displaying dermatological images for monitoring skin conditions.

DE 10 24 0904 describes a device for uniform illumination of object surfaces, especially skin and includes a transparent diffuser between a lamp and the object surface.

### Summary

The present disclosure seeks to propose an improved performance skin diagnosis tool having improved reliability and ease of use, as defined in claim 1.

Embodiments of the disclosure may provide a tool that is robust, reliable, small, and light, and that is capable of providing in a non-invasive, non-traumatic, and non-destructive manner, a diagnosis about the pigmentary status of the skin, e.g. concerning its color, its behavior with regard to the sun, and its chromophores.

According to some embodiments, the device may be portable and handheld, i.e., it may be held in one hand and actuated by one hand.

By means of the diffuser, the skin may be lit in relatively uniform manner, and, if so desired, a component that is less costly than a camera, e.g. a photodiode or a phototransistor, can be used as optical detector.

In addition, the presence of two measuring systems enables a diagnosis to be made that is more complete, with it being possible to combine the information coming from the two measuring systems in order to establish the diagnosis.

The device may also be easy to manipulate because both measuring systems are present within a single appliance. The device may also be easy for the user to hold in the hand.

The diffuser presents a shape that is concave towards the acquisition opening, and advantageously a hemispherical shape, having a greatest diameter that preferably coincides with the acquisition opening, thereby making it possible to diffuse the light in effective manner and avoid unwanted reflections on the skin that are likely to interfere with the measurement.

The lighting system may comprise a plurality of LEDs of different wavelengths, e.g. one or more infra-red (IR) LEDs, one or more ultra-violet (UV) LEDs, and LEDs emitting at red, green, and blue wavelengths respectively.

The different LEDs of a same type may be disposed in uniform manner relative to a sighting axis of the optical detector, e.g. by being uniformly distributed angularly around the sighting axis. The sighting axis may be perpendicular to the plane of the acquisition opening, and may be centered relative to said acquisition opening.

The second measuring system may comprise a detection surface that is sensitive to capacitance, said surface being defined, for example, by at least one row of juxtaposed individual detection cells, e.g. of size not greater than 100 micrometers (µm), as described in US Patent No. 7,402,135, the contents of which are herein incorporated by reference in their entirety.

According to exemplary embodiments of the disclosure, the device further includes a reader for reading a sebum collector device, such a sebum collector device being formed by a strip or by a patch for application to the skin in order to collect sebum therefrom.

The device may include a casing of shape that is generally elongate along a longitudinal axis, and the acquisition opening may be of axis that forms an angle with the longitudinal axis of the casing. The angle between the axis of the acquisition opening and the longitudinal axis of the casing may lie in the range 20° to 60°, for example.

The acquisition opening may be located at the front of the casing.

The casing may carry a screen on its side opposite from its side towards which the acquisition opening faces, e.g. so as to enable the user to observe the screen while the acquisition opening is applied to the skin.

The above-mentioned detection surface for application to the skin may also be situated at the front of the casing, e.g. on the top face of the casing, whereas the acquisition opening may be situated on the bottom face of the casing.

Having both measuring systems near to each other, e.g. at the front of the appliance, may make it easier to handle the device, enabling the user to take a measurement with one of the measuring systems, and then switch easily to the other measuring system, with reduced and/or minimal interaction, e.g., by tilting the device slightly. This may encourage the user to take the measurements from the same region of skin, thereby making the measurements easier and more reliable to process.

The second measuring system may be arranged to take a measurement when the detection surface is moved over the skin, in particular so as to sweep a region already placed in front of the acquisition opening.

The optical detector is housed in the opaque tube that is pointed towards the acquisition opening. The lighting system is located all around the opaque tube.

According to some embodiments, a method for analyzing skin or hair is provided. The method may include obtaining a measurement via the portable skin diagnosis device of the present disclosure and obtaining a non-optical measurement via the portable skin diagnosis device. The method may further include displaying information related to at least one of the measurement and the non-optical measurement.

According further embodiments of the present disclosure the method may also include illuminating the skin via a diffuser and a plurality of LEDs emitting light of different wavelengths. The method may further include LEDs associated with each of the different wavelengths wavelengths of light being illuminated concurrently, with the different wavelengths of light being utilized in succession.

Aside from the structural arrangements set forth above, the disclosure could include a number of other arrangements, such as those explained hereinafter. It is to be understood that both the foregoing description and the following description are exemplary.

### Brief Description of the Drawings

The accompanying drawings, which are incorporated in and constitute a part of this specification, illustrate a number of exemplary features of a non-limiting embodiment of the disclosure and together with the description, serve to explain the principles of the disclosure. In the drawings:
- Figure 1 is a diagrammatic perspective view of an exemplary diagnosis device according to some embodiments of the disclosure;
- Figure 2 is a diagrammatic and fragmentary longitudinal section of the exemplary Figure 1 device;

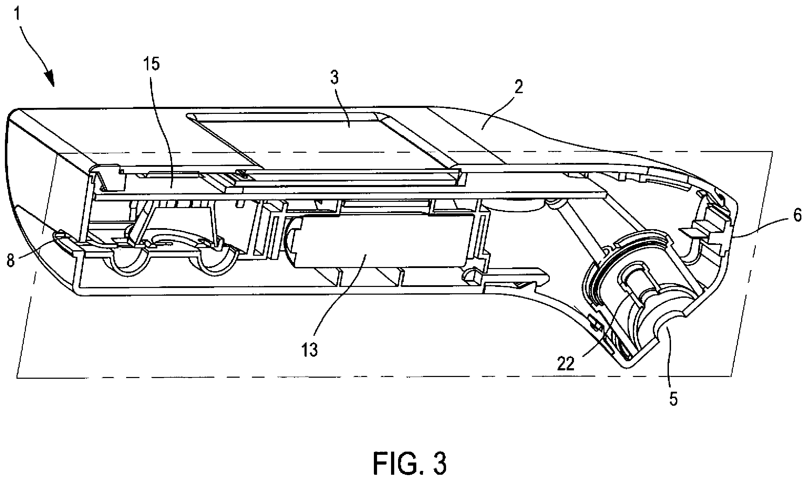

- Figure 3 is another diagrammatic and fragmentary longitudinal section of the exemplary Figure 1 device; and
- Figure 4 is a rear perspective view of an exemplary device according to some embodiments of the present disclosure.

### More Detailed Description

Reference will now be made in detail to exemplary embodiments of the disclosure, examples of which are illustrated in the accompanying drawings.
Wherever possible, the same reference numbers will be used throughout the drawings to refer to the same or like parts. It is to be understood that both the foregoing general description and the following detailed description are exemplary and explanatory only and are not restrictive of the disclosure, as claimed.

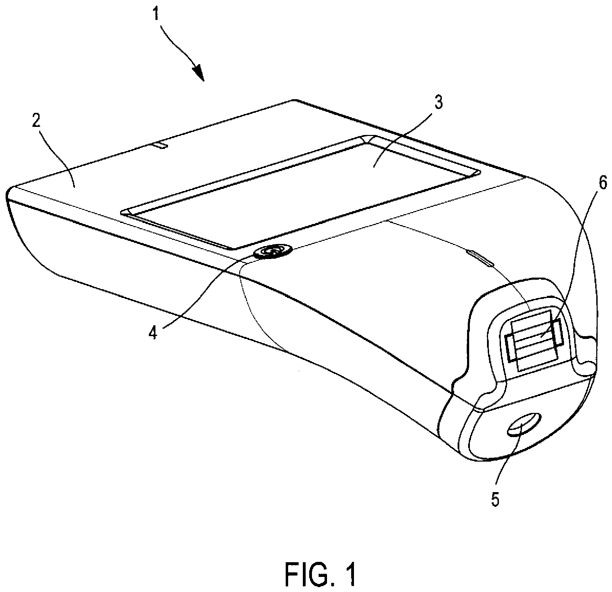

Figure 1 is a diagrammatic perspective view of an exemplary diagnosis device according to some embodiments of the disclosure. Diagnosis device 1 may include a casing 2 having a shape and dimensions, for example, that enable it to be manipulated by hand during use. According to some embodiments, the device may be portable and handheld, i.e., it may be held in one hand and actuated by one hand. As shown, casing 2 may present a shape that is generally elongate along a longitudinal axis X, for example.

Diagnosis device 1 may include a user interface enabling information exchange, for example displaying a diagnosis, images from an atlas, and/or indications about the operation of diagnosis device 1.

According to some embodiments, the user interface may comprise a display, e.g., touch screen 3, that is disposed on top of casing 2, and an ON/OFF button 4.

At its front end, diagnosis device 1 may include an acquisition opening 5 for placing in front of and/or in proximity to the skin to take various optical measurements. Further, a detection surface 6 may be provided, also for placing against the skin to take a non-optical measurement, as described in greater detail below.

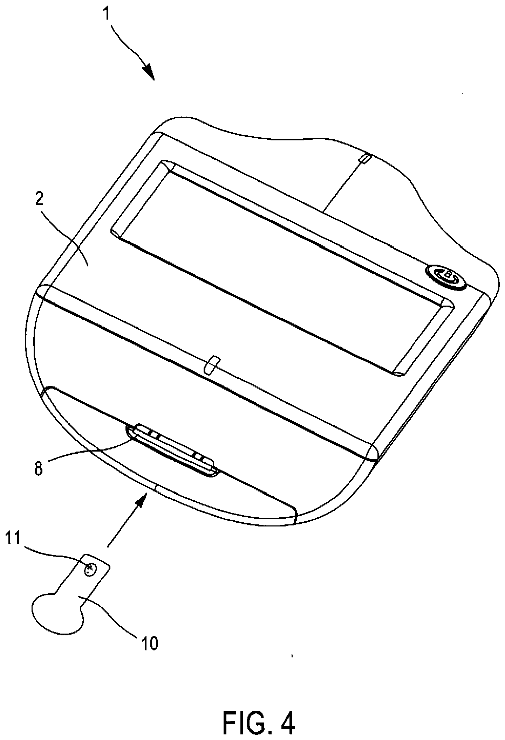

Figure 4 is a rear perspective view of an exemplary device according to some embodiments of the present disclosure. Casing 2 may include a slot 8 for inserting, for example, a collector device 10 (e.g., a sebum collector). Collector device 10 may include a collection surface 11 configured to contact the skin. Collection surface 11 may be defined by materials that, for example, are capable of absorbing sebum and of changing appearance as a function of, for example, the quantity of sebum absorbed.

Casing 2 may also include a slot for inserting a memory card, e.g. a Secure Digital (SD) type memory card, that makes it possible to record and/or store measurements to the memory, and where desired, to load such measurement, and/or device-controlling software into diagnosis device 1.

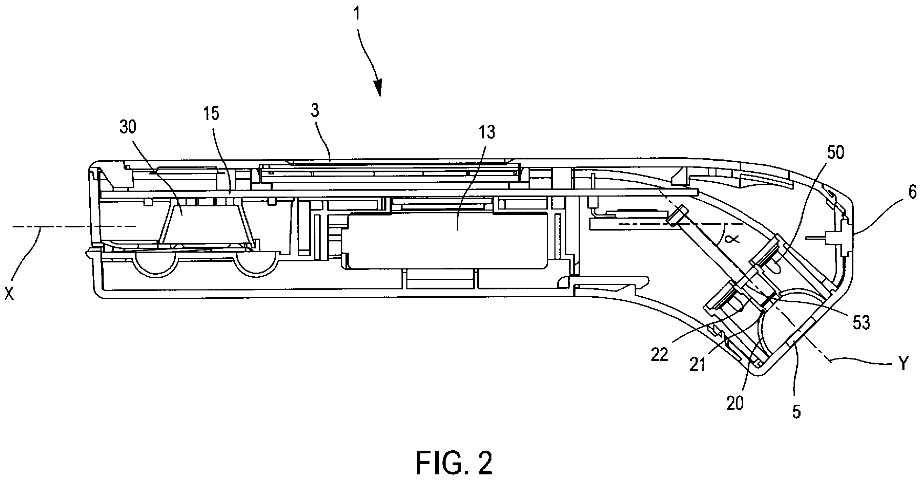

According to some embodiments, a axis Y of acquisition opening 5 and longitudinal axis X may be co-planer, for example, lying in a mid-plane of the casing 2, and forming an angle α (shown at Figure 2) of about 45° between them.

According to some embodiments, acquisition opening 5 may have a circular outline, as shown.

Casing 2 may house a power supply 13 that may include one or more optionally-rechargeable batteries, for example, and an electronic circuit card 15 that may include various electronic components (e.g., a processor and/or related integrated circuits) enabling diagnostic device 1 to operate (e.g., via execution of various commands and functions). In addition, circuit card 15 and/or power supply 13 may provide various functionality for managing the user interface (e.g., touch screen 3). Any suitable combination of electronics may be provided with circuit card 15, for example, a processor may be used to process the measurement data, e.g. an embedded personal computer (PC) type processor.

According to some embodiments, detection surface 6 may face forward, with a normal to said surface being parallel to the longitudinal axis X, for example.

Diagnostic device 1 may include a first measuring system that comprises a diffuser 20, e.g. of hemispherical shape, that is provided at its pole with an opening 21, e.g. of circular shape, in which there is engaged a sighting tube 22. For example, sighting tube may be at its end engaged in diffuser 20. Sighting 22 tube may be provided with a shoulder portion that is suitable for bearing against the outside surface of the diffuser 20.

A lighting system 50 comprising a plurality of LEDs is disposed around sighting tube 22, for example behind an end that is adjacent to the opening 5. The sighting tube comprises an opaque material.

Lighting system 50 illuminates the skin placed behind the acquisition opening 5, the light being transmitted to the skin through diffuser 20. In some embodiments, lighting system 50 may include several groups of LEDs respectively emitting IR, in particular at a wavelength of 830 nanometers (nm), UV, in particular at a wavelength lying in the range 395 nm to 400 nm, red at a wavelength of 636 nm, green at wavelengths of 529 nm and 574 nm, and blue at a wavelength of 430 nm.

According to some embodiments of the present disclosure, each group of LEDs comprises three LEDs that are uniformly distributed angularly around the axis Y.

Diffuser 20 includes a material that is transparent to the wavelengths of light under consideration, e.g. non-doped polymethyl methacrylate (PMMA). The term "transparent" should be understood to mean that the diffuser absorbs less than 20% of the light at the wavelengths under consideration. Diffuser 20 may be for example, transparent to UV, and may further be configured to diffuse light, e.g., translucent and frosted. Lighting through diffuser 20 may enable measurements of skin color more accurately in a D/0 color space.

According to some embodiments, optical system 50 may illuminate the convex outside surface of diffuser 20 over its entire annular region situated around the sighting tube 22.

Before, during, and/or after the first measuring system is in operation, the different groups of LEDs may be powered, for example, in succession to take measurements at the different wavelengths.

The first measuring system may enable measurements of, for example, skin pigmentation and aging.

Lighting successively using the different LEDs may enable obtaining different contributions from pigments of the skin, and in particular of melanin and of hemoglobin.

The use of UV light may enable obtaining the optical response from the surface of the skin only, and, for example, may enable determination of the phototype of the skin. See e.g., "A new device for objective assessment of skin type in Caucasians by violet light reflectance", Rubegni et al., International, Toumal of Cosmetic Science, 2002, 24, 187-193, (describing phototypes).

Further, it may be desirable to include one or more conversion matrices for determining characteristics of the skin from the measurements taken. Such matrices may be stored in, for example, internal memory, an external memory (e.g. a database), or any other suitable location.

Sighting tube 22 may house a photodetector 53, for example, a broadband detector (e.g., a photodiode and/or a phototransistor). Photodetector 53 may be fitted with a lens, for example, that provides photodetector 53 with a narrowed viewing angle, such that the presence of the sighting tube 22 and of the lens may reduce the sensitivity of photodetector 53 to light that is reflected by the concave inside surface of diffuser 20.

Detection surface 6 may be provided with a second measuring system that, in some embodiments, may not be optical. For example, detection surface 6 may comprise a sensor, e.g., a Skinchip® sensor as described in US Patent No. 7 402 135. The sensor may include a strip of, for example, 218 × 8 capacitive detector cells at a cell pitch of 50 µm. One of ordinary skill in the art will recognize that such a sensor may include any desirable sensor for measuring a characteristic, and that the sensor described is exemplary only.

According to some embodiments of the present disclosure, the second measuring system may be used to measure, for example, hydration of the skin.

The reader of the collector device 10 may comprise an inner chamber 30, lighting means, and optical detection means. For example, the reader of the collector device 10 may open to collection surface 11 when collector device 10 is inserted through slot 8. US Patent No. 4,313,393 describes an example of a sebum collector device that may be implemented according to some embodiments of the present disclosure.

Further, optical detection means may operate by reflection for determining the lightness of the collection surface 11. The lighting and detection means may comprise a photodiode and an analog to digital (A/D) converter for sampling light intensity. Such photodiodes may have a wide aperture and may read the lightness of collector device 10 in a more uniform manner.

According to some embodiments of the present disclosure, lighting may be achieved using red LEDs, which may enable disirable sebum-detection sensitivity to be obtained, e.g. two red LEDs emitting with a wide beam angle. Analysis may be performed, for example, through a black, matt sleeve that is provided with an opening that may be smaller than the total size of the collection surface.

According to some embodiments, the reader of the collector device 10 may be arranged to take measurements by transmission, the collection surface in such embodiments being transparent or translucent.

Embodiments of the present disclosure may enable, for example, at a point of sale, characterization of the pigmentary state of the skin (e.g., facial skin). Such characterization may enable, for example study of the effects of a composition on the pigmentation, determination of typologies, and/or recommendation of products and their application methods.

The disclosure is not limited to the embodiment described above. Various modifications could be applied to the device, and in particular other optical and non-optical measuring systems could be provided. For example, a plurality of readers could be provided for sebum collector devices that are associated with different regions of the face, e.g. the cheeks and the forehead. In yet another example, the device could be arranged to display mapped images of wrinkles and/or of sagging/drooping skin.

In some embodiments, diagnostic device 10 could further include a camera, e.g., for measuring pore size.

Where desired, diagnostic device 10 may be arranged to communicate with a portable computer, a router, and/or a computer server, among other things. Such connections may be established via, for example, a wireless connection (e.g. IR, Bluetooth, WiFi, etc).

Throughout the description, including the claims, the term "comprising a" should be understood as being synonymous with "comprising at least one" unless otherwise stated. In addition, any range set forth in the description, including the claims should be understood as including its end value(s) unless otherwise stated. Specific measurement values for described elements should be understood to be within generally accepted manufacturing or industry tolerances, and any use of the terms substantially and/or approximately should be understood to mean falling within such generally accepted tolerances.

Although the present disclosure herein has been described with reference to particular embodiments, it is to be understood that these embodiments are merely illustrative of the principles and applications of the present disclosure. It is therefore to be understood that numerous modifications may be made to the illustrative embodiments and that other arrangements may be devised without departing from the spirit and scope of the present disclosure as defined by the appended claims.

## Claims

1. A portable skin diagnosis device (1) comprising:
• a first measuring system comprising:
• a diffuser (20);
• an acquisition opening (5) for placing in front of the skin;
• a lighting system so as to illuminate the skin in the acquisition opening (5);
• an optical detector (53) that receives the light reflected by the skin placed in the acquisition opening; wherein
• the lighting system (50) is placed behind the diffuser so as to illuminate the skin in the acquisition opening (5) via the diffuser; and
• the optical detector is housed in an opaque tube that is pointed towards the acquisition opening, the lighting system surrounding the opaque tube and the diffuser including a material that is transparent to the wavelengths of the light emitted by the lighting system, the diffuser presenting a shape that is concave towards the acquisition opening (5); and wherein the device further comprises
• a second measuring system that is configured to take a measurement on the skin in a manner that is not optical.

2. A device according to claim 1, the diffuser (20) presenting a shape that is hemispherical.

3. A device according to claim 1 or claim 2, the diffuser (20) presenting a shape that is hemispherical and being provided at its pole with an opening in which is engaged the opaque tube.

4. A device according to any one of claims 1 to 3, the lighting system comprising a plurality of LEDs emitting light at different wavelengths.

5. A device according to claim 4, the lighting system comprising one or more IR LEDs or one or more UV LEDs or LEDs emitting at red, green, and blue wavelengths respectively.

6. A device according to any one of claims 1 to 5, the second measuring system comprising a sensor that is sensitive to capacitance, said sensor comprising at least one row of juxtaposed individual detection cells, each cell having measurements not greater than 100 µm.

7. A device according to any one of claims 1 to 6, including a reader for reading a sebum collector device (10), formed by a strip or a patch for application to the skin in order to collect sebum therefrom.

8. A device according to any one of claims 1 to 7, including a casing of shape that is generally elongate along a longitudinal axis (X), and the acquisition opening being of axis (Y) that forms an angle (α) with the longitudinal axis (X) of the casing, the angle between the axis (Y) of the acquisition opening and the longitudinal axis (X) of the casing lying in the range 20° to 60°.

9. A device according to any one of claims 1 to 8, the second measuring system including a detection surface (6) for application to the skin, both the acquisition opening and the detection surface (6) being situated at the front portion of a casing of the device.

10. A method for analyzing skin or hair, the method comprising:
obtaining a measurement via the portable skin diagnosis device (1) according to any of claims 1 to 9; and
obtaining a non-optical measurement via the portable skin diagnosis device according to any of claims 1 to 9.

11. The method of claim 10, further comprising displaying information related to at least one of the measurement and the non-optical measurement.

12. The method of any of claims 10 and 11, further comprising, illuminating the skin with a plurality of LEDs emitting different wavelengths of light.

13. The method of any of claims 10 to 12, wherein the LEDs associated with each of the different wavelengths of light are illuminated concurrently.

## Patentansprüche

1. Tragbare Hautdiagnoseeinrichtung (1), die Folgendes umfasst:
• ein erstes Messsystem, das Folgendes umfasst:
• einen Diffusor (20);
• eine Erfassungsöffnung (5) zum Anordnen vor der Haut;
• ein Beleuchtungssystem, um die Haut in der Erfassungsöffnung (5) zu beleuchten;
• eine optische Erkennungseinrichtung (53), die das Licht, das durch die Haut, die in der Erfassungsöffnung angeordnet ist, reflektiert wird, empfängt; wobei
• das Beleuchtungssystem (50) hinter dem Diffusor angeordnet ist, um die Haut in der Erfassungsöffnung (5) mittels des Diffusors zu beleuchten; und
• die optische Erkennungseinrichtung in einem lichtundurchlässigen Rohr untergebracht ist, das zu der Erfassungsöffnung weist, das Beleuchtungssystem das lichtundurchlässige Rohr umgibt und der Diffusor ein Material enthält, das für die Wellenlängen des Lichts, das durch das Beleuchtungssystem ausgestrahlt wird, durchlässig ist, wobei der Diffusor eine Form aufweist, die zu der Erfassungsöffnung (5) konkav ist; wobei die Einrichtung ferner Folgendes umfasst:
• ein zweites Messsystem, das konfiguriert ist, eine Messung auf der Haut in einer nicht optischen Weise durchzuführen.

2. Einrichtung nach Anspruch 1, wobei der Diffusor (20) eine halbkugelförmige Form besitzt.

3. Einrichtung nach Anspruch 1 oder Anspruch 2, wobei der Diffusor (20) eine Form aufweist, die halbkugelförmig ist und an ihrem Pol mit einer Öffnung, in die das lichtundurchlässige Rohr eingesetzt ist, versehen ist.

4. Einrichtung nach einem der Ansprüche 1 bis 3, wobei das Beleuchtungssystem mehrere LEDs umfasst, die Licht bei verschiedenen Wellenlängen ausstrahlen.

5. Einrichtung nach Anspruch 4, wobei das Beleuchtungssystem eine oder mehrere IR-LEDs, eine oder mehrere UV-LEDs oder LEDs, die rote, grüne bzw. blaue Wellenlängen ausstrahlen, umfasst.

6. Einrichtung nach einem der Ansprüche 1 bis 5, wobei das zweite Messsystem einen Sensor umfasst, der kapazitätsempfindlich ist, wobei der Sensor mindestens eine Reihe von nebeneinandergestellten einzelnen Detektionszellen umfasst, wobei jede Zelle Abmessungen aufweist, die nicht größer als 100 µm sind.

7. Einrichtung nach einem der Ansprüche 1 bis 6, die eine Lesevorrichtung zum Auslesen einer Talgsammeleinrichtung (10), die durch einen Streifen oder ein Patch zur Anwendung auf die Haut, um Talg davon zu sammeln, enthält.

8. Einrichtung nach einem der Ansprüche 1 bis 7, die ein Gehäuse mit einer Form, die sich im Wesentlichen entlang einer Längsachse (X) erstreckt, und die Erfassungsöffnung, die die Achse (Y) besitzt, die einen Winkel (α) mit der Längsachse (X) des Gehäuses bildet, enthält, wobei der Winkel zwischen der Achse (Y) der Erfassungsöffnung und der Längsachse (X) des Gehäuses im Bereich von 20° bis 60° liegt.

9. Einrichtung nach einem der Ansprüche 1 bis 8, wobei das zweite Messsystem eine Detektionsoberfläche (6) zur Anwendung auf die Haut enthält und sowohl die Erfassungsöffnung als auch die Detektionsoberfläche (6) an dem Vorderseitenabschnitt eines Gehäuses der Einrichtung angeordnet sind.

10. Verfahren zum Analysieren von Haut oder Haaren, wobei das Verfahren Folgendes umfasst:
Erhalten eines Messergebnisses mittels der tragbaren Hautdiagnoseeinrichtung (1) gemäß einem der Ansprüche 1 bis 9; und
Erhalten eines nicht-optischen Messergebnisses mittels der tragbaren Hautdiagnoseeinrichtung gemäß einem der Ansprüche 1 bis 9.

11. Verfahren nach Anspruch 10, das ferner ein Anzeigen von Informationen, die in Beziehung zu der Messung und/oder der nicht-optischen Messung stehen, umfasst.

12. Verfahren nach einem der Ansprüche 10 und 11, das ferner ein Beleuchten der Haut mit mehreren LEDs, die Licht verschiedener Wellenlängen von Licht aussenden, umfasst.

13. Verfahren nach einem der Ansprüche 10 bis 12, wobei die LEDs, die jeder der verschiedenen Wellenlängen von Licht zugeordnet sind, gleichzeitig erleuchtet werden.

## Revendications

1. Dispositif portatif (1) de diagnostic de la peau, comportant :
- un premier système de mesure, comportant :
- un diffuseur (20),
- une ouverture d'acquisition (5) à placer devant la peau,
- un système d'éclairage destiné à éclairer la peau dans l'ouverture d'acquisition (5) ;
- un détecteur optique (53) qui reçoit la lumière réfléchie par la peau placé dans l'ouverture d'acquisition ;
dans lequel
- le système d'éclairage (50) est placé derrière le diffuseur de sorte à éclairer la peau dans l'ouverture d'acquisition (5) à travers le diffuseur, et
- le détecteur optique est logé dans un tube opaque qui est orienté vers l'ouverture d'acquisition, le système d'éclairage entourant le tube opaque et le diffuseur incluant un matériau qui est transparent aux longueurs d'onde de la lumière émise par le système d'éclairage, le diffuseur présentant une forme qui est concave vers l'ouverture d'acquisition (5) ; et dans lequel le dispositif comprend en outre
- un second système de mesure qui est configuré pour réaliser une mesure non optique sur la peau.

2. Dispositif selon la revendication 1, le diffuseur (20) présentant une forme hémisphérique.

3. Dispositif selon la revendication 1 ou la revendication 2, le diffuseur (20) présentant une forme qui est hémisphérique et étant pourvu à son pôle d'une ouverture dans laquelle s'engage le tube opaque.

4. Dispositif selon l'une quelconque des revendications 1 à 3, le système d'éclairage comportant plusieurs LEDs émettant de la lumière à différentes longueurs d'onde.

5. Dispositif selon la revendication 4, le système d'éclairage comportant une ou plusieurs LEDs IR ou une ou plusieurs LEDs UV ou des LEDs émettant respectivement à des longueurs d'onde rouge, verte et bleue.

6. Dispositif selon l'une quelconque des revendications 1 à 5, le deuxième système de mesure comportant un capteur qui est sensible à la capacitance, ledit capteur comportant au moins une rangée de cellules de détection individuelles juxtaposées, chaque cellule étant de dimension inférieure ou égale à 100 µm.

7. Dispositif selon l'une quelconque des revendications 1 à 6, comportant un lecteur pour lire un dispositif (10) de collecte de sébum, formé d'une bande ou d'une pastille à appliquer sur la peau pour en collecter le sébum.

8. Dispositif selon l'une quelconque des revendications 1 à 7, comportant un boîtier de forme généralement allongée selon un axe longitudinal (X) et l'ouverture d'acquisition étant d'axe (Y) faisant un angle (α) avec l'axe longitudinal (X) du boîtier, l'angle entre l'axe (Y) de l'ouverture d'acquisition et l'axe longitudinal (X) du boîtier étant compris entre 20 et 60°.

9. Dispositif selon l'une quelconque des revendications 1 à 8, le deuxième système de mesure comportant une surface de détection (6) à appliquer sur la peau, l'ouverture d'acquisition et la surface de détection (6) étant toutes deux situées à l'avant d'un boîtier du dispositif.

10. Procédé d'analyse de la peau ou des cheveux, le procédé comprenant :
l'obtention d'une mesure par le biais du dispositif portatif (1) de diagnostic de la peau selon l'une quelconque des revendications 1 à 9 ; et
l'obtention d'une mesure non optique par le biais du dispositif portatif (1) de diagnostic de la peau selon l'une quelconque des revendications 1 à 9.

11. Procédé selon la revendication 10, comprenant en outre l'affichage d'informations relatives à au moins une de la mesure et de la mesure non optique.

12. Procédé selon l'une quelconque des revendications 10 et 11, comprenant en outre l'éclairage de la peau avec une pluralité de LEDs émettant différentes longueurs d'onde de lumière.

13. Procédé selon l'une quelconque des revendications 10 à 12, dans lequel les LEDs associées à chacune des différentes longueurs d'onde de lumière sont éclairées simultanément.
